# EUROPÄISCHE PATENTSCHRIFT

(11) **EP 2 326 307 B1**
(45) Veröffentlichungstag und Bekanntmachung des Hinweises auf die Patenterteilung: **28.10.2015**
(21) Anmeldenummer: 09776893.1
(22) Anmeldetag: 30.06.2009
(51) Int. Cl.: H04R 5/00, A61K 9/16

(54) **VERFAHREN ZUR HERSTELLUNG KRISTALLINER WIRKSTOFF-MIKROPARTIKEL**
METHOD FOR PRODUCING CRYSTALLINE ACTIVE INGREDIENT MICROPARTICLES
PROCÉDÉ DE FABRICATION DE MICROPARTICULES DE SUBSTANCE ACTIVE CRISTALLINES

(30) Priorität: 08.08.2008 DE 102008037025
(43) Veröffentlichungstag der Anmeldung: 01.06.2011
(60) Teilanmeldung: 15170525.8
(73) Patentinhaber: Jesalis Pharma GmbH, 07745 Jena (DE)
(72) Erfinder: GRAWE, Detlef, 99510 Kleinromstedt (DE); GLIESING, Sabine, 07743 Jena (DE); EILERS, Robert, 07745 Jena (DE)
(74) Vertreter: Geyer, Fehners & Partner
(86) Internationale Anmeldenummer: PCT/EP2009/004724
(87) Internationale Veröffentlichungsnummer: WO 2010/015298

(56) Entgegenhaltungen:
- EP-A1- 1 944 018
- EP-A2- 0 454 138
- WO-A1-01/14036
- US-A1- 2003 175 187

## Beschreibung

Die Erfindung, definiert durch die Ansprüche, bezieht sich auf ein Verfahren zur Herstellung kristalliner Mikropartikel eines Wirkstoffs, bei dem der Wirkstoff aus einer Suspension aus Primärpartikeln, Lösungsmittel, Nicht-Lösungsmittel und inerten Formkörpern kristallisiert wird. Ferner bezieht sich die Erfindung auf ein Verfahren zur Herstellung einer Wirkstoffpartikel-Festkörperform und die nach diesen Verfahren erhältlichen Wirkstoff-Mikropartikel und Arzneimittel-Festkörperformen, die die Mikropartikel enthalten.

Für eine hohe Wirksamkeit und Bioverfügbarkeit von Arzneistoffen kann es erforderlich sein, in sehr kurzer Zeit zu hohen Serumspiegeln von Wirkstoffen zu gelangen, wofür möglichst schnell freisetzende Arzneiformen benötigt werden. Die schnelle Freisetzung wird jedoch häufig durch die schlechte Wasserlöslichkeit von Wirkstoffen erschwert.

Die Freisetzungskinetik und damit die Bioverfügbarkeit von Arzneistoffen ist neben den Zerfallseigenschaften der Arzneiform in erster Linie von der Partikelgröße, der Partikelgrößenverteilung und der Kristallinität des Wirkstoffes abhängig. Zur Beschleunigung der Freisetzung von schwer löslichen Wirkstoffen werden diese in der Regel mikronisiert, d.h. die festen Wirkstoffe werden auf Partikelgrößen im Mikrometerbereich zerkleinert. Üblicherweise erfolgt dies nach dem Strahlmühlenverfahren.

Die Mikronisierung, die mit ihr erzeugten Mikronisate und ihre Verwendung in pharmazeutischen Arzneiformen weisen jedoch Nachteile im Hinblick auf unerwünschte Amorphisierungen und unter dem Gesichtspunkt der pharmazeutischen Verarbeitbarkeit auf (Thilbert and Tawashhi: "Micronization of pharmaceutical solids", MML-Series, 1999, Bd.1, Kap.1, S. 328-347). Die Amorphisierung ist umso ausgeprägter, d.h. die Kristallinität umso geringer, je feiner die Körnung, also je höher der zur Mahlung erforderliche Energieeintrag ist. Demzufolge weisen gerade sehr feinkörnige Mikronisate, wie sie für schnelle Freisetzungen und pulmonale Applikationen benötigt werden, z. B. mit einer durchschnittlichen Korngröße von 1-1,5 µm, einen hohen amorphen Anteil auf, der für bestimmte Wirkstoffen bei 5-20% und höher liegen kann, d. h. die Kristallinität beträgt 80-95 Gew.-% oder niedriger.

Die teilweise Amorphisierung bei Mikronisierungen ist mit einer chemischen und physikalischen Destabilisierung des Wirkstoffes verbunden. Nachfolgende Agglomerationen und Rekristallisationen können ferner die Freisetzung aus der Arzneiform beeinträchtigen. Diese Nachteile treten umso deutlicher hervor, je feiner die Körnung im Sinne einer schnellen Freisetzung angestrebt wird. Daher können bei Mikronisaten sowohl als Wirkstoff als auch in der Arzneiform in Kontakt mit den Hilfsstoffen Stabilitätsprobleme auftreten. Ferner neigen Mikronisate zu einer starken Aufladung, Staubbildung sowie zu schlechten Schütt- und Rieseleigenschaften, die die Verarbeitung zur Arzneiform nur in speziellen, kostenaufwendigen Verfahren, wie z.B. der Wirbelschichtfeuchtgranulation ermöglichen.

Für sehr schnelle Freisetzungen sind bei schlecht löslichen Wirkstoffen Partikelgrößen erforderlich, die über Mikronisierverfahren nicht oder nur begleitet von starken Amorphisierungen zu erreichen sind. Die Agglomeration und Krustenbildung schon während des Mahlvorganges können eine Vermahlung zusätzlich erschweren. Darüber hinaus weisen Mikronisate in der Regel eine relativ breite Korngrößenverteilung auf. Wünschenswert ist für eine schnelle Freisetzungskinetik aber eine enge Korngrößenverteilung, bei der sowohl die Nanoanteile als auch die Anteile der größeren Partikel minimiert werden.

Mikronisate können über Präformulierungen so mit Hilfsstoffen kombiniert werden, daß einige nachteilige Eigenschaften, wie schlechte Verarbeitbarkeit, verbessert werden. In der DE 103 25 989 A1 sind Wirkstoff-Hilfsstoff-Mikropellets beschrieben, die auf Basis von Mikronisaten durch Wirbelschichtgranulation hergestellt werden. Diese Technologie ist jedoch untrennbar mit den oben aufgeführten Nachteilen, die sich aus dem destruktiven, energiereichen Mahlprozeß und der erreichbaren Körnung ergeben, verknüpft. Außerdem sind derartige grobkörnige Granulate für Low-Dose- und Ultra-Low-Dose-Formulierungen wegen der erforderlichen Gehaltsgleichförmigkeit des Wirkstoffes nicht geeignet.

Sehr feine kristalline Partikel können durch Naßmahlverfahren mit und ohne Zusatzstoffe, wie beispielsweise Tenside, Hydrokolloide oder Zucker, in Kugelmühlen oder Hochdruckhomogenisatoren erzeugt werden, wie beispielsweise in der US 5 145 684, US-A-5 091 187, US-A-5 858 410 beschrieben. Hier werden durch einen extrem hohen spezifischen Energieeintrag grobe Partikel bis auf Nanogröße zermahlen. Dieser Prozeß benötigt jedoch viel Zeit und oftmals ist ein vielfacher Durchlauf der Suspension durch den Mühlenraum erforderlich. Neben dem hohen apparativen Aufwand und möglicher Amorphisierungen sind Kontaminationen durch Abrieb und Verschleiß der Mahlvorrichtung weitere Nachteile.

Zur Vermeidung dieser Nachteile können Wirkstoffe zur Erzielung schneller Freisetzungen vollständig amorph sprühgetrocknet oder molekulardispers in hydrophile, polymere Hilfsstoffe eingebettet werden (E. Nürnberg in "Darstellung und Eigenschaften pharmazeutisch relevanter Sprühtrocknungsprodukte", Acta Pharm. Techn., 26(1), S. 40-67, 1980). Mit konkreten Wirkstoffen und auf spezifische pharmazeutische Applikationen bezogen, ist dies in der EP 04103837 A1 beschrieben. Diese Formen haben jedoch den Nachteil, daß die guten Löslichkeitseigenschaften durch erhebliche Risiken in der chemischen Stabilität und bei entsprechend hoher Beladung auch in der physikalischen Stabilität erkauft werden.

Ein Sonderfall der molekularen Einbettung zur Beschleunigung der Freisetzung ist die Bildung von Einschlußkomplexen mit Cyclodextrinen. Dieses Verfahren ist jedoch an eine zur Komplexierung geeigneten Molekülgeometrie gebunden und daher nicht universell einsetzbar.

Eine weitere Technologie besteht im Aufsprühen einer organischen Wirkstofflösung auf einen Träger, wie beispielsweise in der DE 19652196 A1 beschrieben. Dies kann während der Wirbelschichtgranulation zur Herstellung des Tablettengranulates erfolgen oder aber über Sprühtrocknung als hochbeladene Präformulierung. Die Nachteile dieser Technologien bestehen in der häufigen vollständigen bzw. partiellen Amorphisierung beim Sprühtrocknen von Wirkstofflösungen oder aber in der Erzeugung einer instabilen kristallinen Modifikation. Auch ist bei den beschriebenen hochbeladenen Präformulierungen eine schnelle Freisetzung nicht automatisch gegeben, da sich die Wirkstoffschichten auf dem mikronisierten Carrier nicht schneller lösen als vergleichbare Wirkstoffmikronisate. Diese Technologie hat ihre Stärken eher in der Erzielung einer guten Gehaltsgleichförmigkeit für Low-Dose- bzw. Ultra-Low-Dose-Formulierungen und einer besseren Verarbeitung schlecht mikronisierbarer Wirkstoffe. Außerdem erhöht die Verwendung brennbarer Lösungsmittel den technologischen Aufwand im Herstellprozeß.

Eine Alternative zur Mikronisierung und zur Verwendung von amorphen bzw. kristallinen sprühformulierten Wirkstoff-Hilfsstoff-Kombinationen ist die Herstellung von Kristallen mit einer dem geforderten Freisetzungsverhalten angepaßten Korngrößenverteilung durch Kristallisation in einer Vorrichtung zum Naßmahlen mit anschließender Temperaturoszillation, wie in der EP 1497308 B1 beschrieben. Die nach diesem Verfahren erhältlichen Partikel sind zwar kristallin, erreichen jedoch in der Regel nicht die Partikelgröße, die für eine sehr schnelle Freisetzung erforderlich ist. So wird in der Regel nicht einmal die durchschnittliche Korngröße von Mikronisaten erreicht. Hinzu kommt, daß gerade bei der Kristallisation von extrem feinen Partikeln aus stark übersättigten Lösungen die feine Primärpartikelkörnung durch eine überlagerte Agglomeration wieder verloren geht. Für sehr schnell freisetzende Formen stößt diese Technologie daher an ihre Grenzen.

In der DE 10214031 A1 wird ein Fällungsverfahren zur Herstellung von Mikro- und Nanoteilchen für schnelllösliche und auch pulmonale Arzneiformen beschrieben. Der Wirkstoff wird in einem Lösungsmittel gelöst und anschließend mit einem Nonsolvent unter Anwesenheit eines Kristallwachstumsinhibitors ausgefällt. Der Nachteil dieses Verfahrens besteht darin, daß auch hier durch den schnellen Phasentransfer, analog der Sprühtrocknung, in der Regel amorphe bzw. metastabile Phasen erzeugt werden. Dies betrifft vor allem schlecht kristallisierbare Wirkstoffe mit hoher konfigurativer Entropie, wo die stabile Kristallform kinetisch benachteiligt ist. Durch den Kristallwachstumsinhibitor wird ein Phasenübergang in die stabile Modifikation während des Herstellungsprozesses zusätzlich erschwert oder gar verhindert. Damit sind unwägbare Stabilitätsprobleme in der Arzneiform verbunden. Ein weiter Nachteil ist, daß trotz Inhibition des Wachstums die fällbare Korngröße stark von den physiko-chemischen Eigenschaften des Wirkstoffs abhängt und diese für eine sehr schnelle Freisetzung nicht immer ausreichend fein ist.

Weitere Verfahren zur Herstellung von Suspensionen feinkörniger Wirkstoffpartikel durch Prezipitation werden in der US-A-5 389 382, der US-A-2005 0 139 144 und der US-A-2002 0 127 278 beschrieben. Hier wird das Wachstum der Kristalle jedoch durch die Fällungsbedingungen (z.B. Mischungsverhältnis), die Anwesenheit von Stabilisatoren bzw. durch entsprechende energetische Nachbehandlung im Hochdruckhomogenisator begrenzt.

Ein anderes Fällungsverfahren zur Herstellung von Wirkstoff-Nanopartikeln wird in der DE 10 2005 053 862 A1 beschrieben. Hier wird kurz vor oder in einer volumenmäßig kleinen, aber hochenergetischen Zone (Ultraschallzelle, Scherspalt eines Hochdruckhomogenisators, Rotor-Stator-Spalt einer Kolloidmühle) der gelöste Wirkstoff mit einem Nonsolvent gemischt und ausgefällt. Diese als Fällungsreaktoren modifizierten Hochenergie-Nassmahleinrichtungen arbeiten nach dem Durchlaufprinzip kontinuierlich. Im Unterschied zum reinen Mahlprozeß kann diese Kombination Partikelgrößen bis weit in den Nanobereich produzieren. Auch dieses Verfahren ist jedoch mit dem Nachteil verbunden, daß ein hoher technologischer Aufwand erforderlich ist und häufig Kontaminationsprobleme (z.B. mit Sonotrodenmaterial) auftreten. Für schwer oder langsam kristallisierende Wirkstoffe, oder für solche, die instabile, z.B. amorphe Zwischenphasen durchlaufen, sind diese Anordnungen nicht geeignet. Somit ist dieses Verfahren nur für hinreichend schnell kristallisierende organische Verbindungen geeignet.

In der US 2003/0175187 A1 ist ein Verfahren und eine Apparatur zum Kristallisieren einer Substanz, insbesondere eines inhalierbaren Medikaments, beschrieben. Das Verfahren umfasst die Schritte, dass (a) eine Substanz in einem Lösungsmittel gelöst wird, um eine Lösung der Substanz zu erzeugen, (b) ein segmentierter Strom erzeugt wird, der aus alternierenden Segmenten der Lösung der Substanz und dem Transportmedium besteht, (c) der segmentierte Strom in eine Verweilstrecke eingebracht wird, (d) die Verweilstrecke abgekühlt wird, um eine Kristallisation der Substanz in den Segmenten der Lösung in dem segmentierten Strom zu veranlassen und (e) die Kristalle des Materials von dem segmentierten Strom abgetrennt werden.

In der WO 01/14036 A1 wird ein Verfahren zum Herstellen von Arzneimittelpartikeln beschrieben, umfassend das Auflösen des Arzneimittels in einem Lösungsmittel, das Erzeugen von einem oder mehreren Strömen der Arzneimittellösung und das Inkontaktbringen dieser Ströme mit einem oder mehreren Strömen von Antilösungsmittel, um einen Bereich von turbulentem Vermischen zu erzeugen, in dem eine schnelle Ausfällung von Arzneimittelkristallen stattfindet, wobei die relative Geschwindigkeit der Ströme 50 m/s übersteigt und das Verhältnis der Volumenströme von Antilösungsmittel zu Medikamentenlösung 2:1 überschreitet.

Ein weiteres Verfahren zur Herstellung von Mikro- und Nanopartikeln ist die Fällung unter Verwendung von überkritischem Kohlendioxid (Kerc, at al: "Micronization of Drugs using supercritical carbon dioxide", Int. J. Pharm. 182, 1999, 33-39 und in Steckel, at al: "Micronizing of steroids for pulmonary delivery by supercritical carbon dioxide", Int. J. Pharm. 152, 1997, 99-110). Diese Verfahren sind jedoch wegen des Umgangs mit überkritischen Gasen mit einem sehr hohen apparativen und technologischen Aufwand verbunden.

Ein grundsätzlicher Nachteil der oben genannten Sprühtrocknungs- und Fällungsverfahren liegt in ihrer Natur begründet. Die meisten Wirkstoffe haben polymorphen Charakter, d.h. sie sind in der Lage, in mehr als einer Kristallmodifikation auszukristallisieren. Für pharmazeutische Anwendungen wird jedoch, bis auf wenige Ausnahmen, die thermodynamisch stabilste Kristallmodifikation bevorzugt. Nach den Ostwald'schen Rule of Stages entstehen aus Lösungen, vor allem bei schneller Prozeßkinetik, bevorzugt die energiereicheren, also weniger stabilen Modifikationen, amorph-kristalline Mischformen oder sogar die reine amorphe Form, wie z.B. regelmäßig bei der Sprühtrocknung von Lösungen. Phasenumwandlungen in die stabile Modifikation sind, wenn sie denn überhaupt in vertretbaren Zeiträumen stattfinden, schwierig zu kontrollieren. Da Fällungsverfahren aufgrund der hohen Übersättigungen naturgemäß extrem schnelle Phasenübergangsprozesse sind, können sich hieraus Probleme in der reproduzierbaren Herstellung einer thermodynamisch stabilen Kristallform ergeben, zumal diese Transformation, die sich mit dem Fällungsprozeß überlagern oder auch später stattfinden kann, die einmal erreichte Korngröße erheblich beeinträchtigen kann.

Eine Aufgabe der vorliegenden Erfindung ist es daher, die Nachteile des Standes der Technik zu überwinden und ein Verfahren bereitzustellen, das mit geringem technischen Aufwand Wirkstoffpartikel im Mikrometerbereich liefert, die hoch kristallin sind, in der stabilsten Kristallmodifikation vorliegen und eine sehr enge Partikelgrößenverteilung aufweisen, so daß sie für sehr schnelle und vollständige Arzneimittelfreisetzungen geeignet sind. Weiter ist es eine Aufgabe der vorliegenden Erfindung, ein Verfahren zur Herstellung einer schnell freisetzenden Wirkstoffpartikel-Festkörperform bereitzustellen. Der vorliegenden Erfindung liegt auch die Aufgabe zugrunde, die nach diesen Verfahren erhältlichen, kristallinen Wirkstoff-Mikropartikel und eine zugehörige Festköperform bereitzustellen.

Diese Aufgabe wird gelöst durch ein Verfahren zur Herstellung kristalliner Wirkstoff-Mikropartikel, umfassend die Schritte, daß aus Primärpartikeln des Wirkstoffs, einer Lösung des Wirkstoffs, Nicht-Lösungsmittel für den Wirkstoff und inerten Formkörpern, mit einer maximalen Ausdehnung von 0,1 - 2 mm, eine Suspension hergestellt wird, die Suspension durchmischt wird, wobei der Wirkstoff aus der Suspension auskristallisiert, der Wirkstoff in Form von Produktpartikeln abgetrennt wird und die Produktpartikel anschließend getrocknet werden, wobei die Primärpartikel und die Produktpartikel kristallin sind, in der stabilsten Kristallmodifikation des Wirkstoffs vorliegen, eine Kristalloberfläche von 3 - 10 m²/g aufweisen und die Größenverteilung der Primär- und Produktpartikel, bestimmt durch Laserbeugung, jeweils d₅₀ = 1 - 2 µm, d₉₉ < 6 µm und d₁₀₀ < 12 µm beträgt, wobei sich diese d-Werte auf Volumenprozente der Partikel beziehen.

Das erfindungsgemäße Verfahren liefert überraschenderweise weitgehend unabhängig von den physiko-chemischen Eigenschaften des Wirkstoffs kristalline Mikropartikel, die eine sehr enge Partikelgrößenverteilung und eine hohe Kristalloberfläche aufweisen und in der stabilsten Kristallmodifikation vorliegen. Dadurch sind die Wirkstoff-Mikropartikel für schnell freisetzende Arzneimittelformen hervorragend geeignet.

Die Aufgabe wird weiter gelöst durch ein Verfahren zur Herstellung einer Wirkstoffpartikel-Festkörperform, bei dem in dem oben beschriebenen Verfahren die abgetrennten Produktpartikel vor dem Trocknen zusätzlich mit einem hydrophilen, pharmazeutisch annehmbaren Hilfsstoff versetzt werden. Mit diesem Verfahren ist eine Wirkstoffpartikel-Festkörperform erhältlich, die einen hydrophilen Hilfsstoff und die kristallinen Substanz-Mikropartikel umfaßt.

Die Aufgabe wird auch durch die nach den oben beschriebenen Verfahren erhältlichen Wirkstoff-Mikropartikel und Wirkstoffpartikel-Festkörperformen gelöst.

Die Wirkstoffpartikel-Festkörperform weist den Vorteil auf, daß sie bei Kontakt mit Wasser, weitgehend unabhängig von der Korngröße der Festkörperform, den Wirkstoff innerhalb weniger Minuten ohne Aggregation als singuläre Partikel freisetzt. Ferner besitzt die Festkörperform sehr gute pharmazeutische Verarbeitungseigenschaften, da sie gröber als übliche Mikronisate ist, eine bessere Rieselfähigkeit aufweist und sich nicht elektrostatisch auflädt, so daß sie insgesamt sehr wirtschaftlich zu Arzneiformen weiterverarbeitet werden kann, z.B. durch Trockenmischverfahren in der Tablettenherstellung. Insbesondere ist die Wirkstoffpartikel-Festkörperform besonders für Low Dose und Ultra Low Dose-Anwendungen geeignet.
Fig. 1 zeigt eine lichtmikroskopische Aufnahme der Wirkstoffpartikel-Festkörperform.
Fig. 2-5 zeigen lichtmikroskopische Aufnahmen von zeitlich aufeinanderfolgenden Phasen des Freisetzungsprozesses bei Kontakt der Wirkstoffpartikel-Festkörperform mit Wasser. Fig. 2 und Fig. 3 zeigen den partikulären Zerfall unmittelbar nach Kontakt mit Wasser, Fig. 4 und Fig. 5 zeigen den Zerfall in die einzelnen kristallinen Mikropartikel nach dreiminütigem Kontakt mit Wasser.
Fig. 6 zeigt die Freisetzung von Drospirenon aus der Wirkstoffpartikel-Festkörperform im Vergleich zu einem konventionellen Mikronisat.

Unter dem Begriff "Wirkstoff" wird im Sinne der Erfindung ein pharmazeutischer Wirkstoff verstanden, d. h. ein Stoff, der eine physiologische Wirkung entfaltet, wenn er in hinreichender Menge vom Körper eines Lebewesens, insbesondere eines Säugetiers, vorzugsweise eines Menschen, absorbiert wird.

Bei dem erfindungsgemäßen Verfahren wird der zu kristallisierende Wirkstoff in einem geeigneten Lösungsmittel oder einer Mischung aus Lösungsmitteln gelöst und mit einem Nicht-Lösungsmittel für den Wirkstoff sowie Primärpartikeln des Wirkstoffs und inerten Formkörpern vermischt. Aus der erhaltenen Kristallisationssuspension kristallisiert der Wirkstoff infolge der Übersättigung in Form von Produktpartikeln aus. Durch die angebotene Kristalloberfläche der Primärpartikel und die zusätzliche Oberfläche der anwesenden Formkörper sowie ein partielles Zerbrechen der Wirkstoffpartikel (Primär- und Produktpartikel) bei ihrer Kollision mit den inerten Formkörpern infolge der Durchmischung werden überraschenderweise Wirkstoff-Produktpartikel erhalten, die kristallin sind, eine sehr enge Partikelgrößenverteilung aufweisen und im gleichen Größenbereich wie die eingesetzten Primärpartikel liegen. Ferner liegen die nach dem erfindungsgemäßen Verfahren erhältlichen Produktpartikel in der stabilsten Kristallmodifikation des Wirkstoffs vor, worunter die thermodynamisch stabilste Kristallmodifikation verstanden wird.

Die Partikelgrößenverteilung (Korngrößenverteilung) wird mit Hilfe einer üblichen Laserbeugung gemessen, die eine Verteilungskurve der Partikelgrößen liefert (System Sympatec Helos, Trockendispergierer Rodos). dₓ bedeutet, daß x Volumenprozent (Vol.-%) der Partikel einen Durchmesser aufweisen, der kleiner als der angegebene Wert ist. Bei einem d₅₀-Wert von 1 µm haben somit 50 Vol.-% der Partikel einen Durchmesser kleiner als 1 µm (Mikrometer). Wenn der d₁₀, d₉₉ oder d₁₀₀ 2 µm beträgt, weisen 10, 99 oder 100 Vol.-% der Partikel einen Durchmesser kleiner als 2 µm auf.

Bei dem erfindungsgemäßen Verfahren werden Primärpartikel eingesetzt und Produktpartikel erhalten, deren Größenverteilung die Bedingungen d₅₀ = 1 - 2 µm, d₉₉ < 6 µm und d₁₀₀ < 12 µm erfüllt. Dies bedeutet, daß 50 Vol.-% der eingesetzten und der nach dem Verfahren erhältlichen Partikel einen Durchmesser unterhalb von 1 bis 2 µm, 99 Vol.-% der Partikel einen Durchmesser unterhalb von 6 µm und 100 Vol.-% der Partikel einen Durchmesser kleiner als 12 µm aufweisen. Der d₉₉-Wert von kleiner 6 µm und der d₁₀₀-Wert von kleiner 12 µm zeigt, daß bezogen auf den d₅₀-Wert ein nur sehr geringer Anteil grober Partikel vorliegt. In einer bevorzugten Ausführungsform ist d₅₀ = 1,2 - 1,8 µm, d₉₉ < 6 µm und d₁₀₀ < 12 µm. Besonders bevorzugt ist d₁₀₀ < 8 µm. Bevorzugt ist es ferner, daß auch die Nanoanteile der nach dem Verfahren erhältlichen kristallinen Mikropartikeln minimiert sind. Bevorzugt ist daher d₂₀ < 0,8 µm, insbesondere 0,6 - 0,75 µm. Weiter bevorzugt ist d₁₀ < 0,7 µm, besonders bevorzugt 0,35 - 0,65 µm. Ferner ist es bevorzugt, daß d₅ < 0,4 µm ist, besonders bevorzugt 0,25 - 0,35 µm. Die obigen Größenverteilungen beziehen sich jeweils unabhängig voneinander auf die Primär- und die Produktpartikel. Die Werte für d₂₀, d₁₀ und d₅ können einzeln oder in Kombination mit den oben angegebenen Werten für d₅₀, d₉₉ und d₁₀₀ vorliegen. Beispielsweise ist es bevorzugt, daß die Größenverteilung d₁₀ = 0,35-0,65 µm, d₅₀ = 1 - 2 µm und d₁₀₀ < 8 µm beträgt.

Ein weiterer Vorteil des erfindungsgemäßen Verfahrens ist die hohe Kristallinität der erhältlichen Produktpartikel. Unter hochkristallinen oder kristallinen Partikeln werden im Sinne der Erfindung Partikel verstanden, die keine oder nur geringe amorphe Anteile enthalten und damit überwiegend kristallin sind. Bevorzugt ist die Kristallinität der Produktpartikel größer als die von Mikronisaten. Insbesondere ist die Kristallinität, d.h. der kristalline Anteil ≥ 98 Gew.-%. Bevorzugt ist die Kristallinität der Produktpartikel ≥ 99 Gew.-% und besonders bevorzugt ist es, daß die Kristallinität ≥ 99,9 Gew.-% beträgt.

Die Kristallinität wird mittels Röntgenpulverdiffraktometrie (XRPD) ermittelt. Dabei werden die integralen Intensitäten im Röntgendiffraktogramm nach Untergrundkorrektur mit denen eines Referenzmaterials verglichen. Dieses Referenzmaterial kann beispielsweise ein konventionelles Mikronisat sein oder ein Produkt nach einem anderen Herstellungsverfahren mit bekannter Kristallinität und vergleichbarer Körnung. Damit ist die Kristallinität eine relative Größe, die den Ordnungsgrad im Vergleich zu einer Referenzprobe charakterisiert. Das Verhältnis der integralen Reflexintensitäten von Partikelprobe und Referenzprobe ist daher der quantitative Ausdruck für deren Kristallinitätsunterschied.

Vorzugsweise ist auch die Kristallinität der Primärpartikel größer als die von Mikronisaten. Insbesondere beträgt bei den Primärpartikeln die Kristallinität ≥ 98 Gew.-%, bevorzugt ≥ 99 Gew.-%, besonders bevorzugt ≥ 99,9 Gew.-%. Bevorzugt werden die Produktpartikel aus einer vorangegangenen Herstellung als Primärpartikel eingesetzt, insbesondere aus einer vorangegangenen Herstellung nach dem erfindungsgemäßen Verfahren. Daher weisen die Primärpartikel in einer bevorzugten Ausführungsform die gleichen Eigenschaften wie die Produktpartikel auf, d. h. Primär- und Produktpartikel sind identisch.

Die Oberfläche der eingesetzten Primärpartikel und der nach dem Verfahren erhältlichen Kristalle beträgt 3 - 10 m²/g, bevorzugt 4 - 8 m²/g. Durch die so bei der Kristallisation angebotene, sehr große Kristalloberfläche wird eine hohe Übersättigung im System aus Lösungsmittel und Nicht-Lösungsmittel auch bei langsam kristallisierenden Wirkstoffen schnell in den metastabilen Bereich abgebaut und so die erneute Keimbildung unterdrückt. Der zugeführte, gelöste Wirkstoff kristallisiert dadurch vornehmlich auf vorhandene Kristalloberflächen.

In einer bevorzugten Ausführungsform des erfindungsgemäßen Verfahrens werden die Primärpartikel des Wirkstoffs in Form einer Suspension eingesetzt. Insbesondere hat es sich als günstig erwiesen, die Primärpartikel und die inerten Formkörper in einer Suspension aus Lösungsmittel und Nicht-Lösungsmittel vorzulegen und die Lösung des Wirkstoffs und das Nicht-Lösungsmittel zuzugeben.

Die Produktpartikel können mit üblichen Verfahren abgetrennt werden, beispielsweise durch Filtration. Bevorzugt wird aus dem Kristallisationsansatz eine Suspension von Produktpartikeln entnommen. In einer bevorzugten Ausführungsform umfasst das Verfahren zur Herstellung kristalliner Wirkstoff-Mikropartikel daher die Schritte, daß zu einer Suspension von Primärpartikeln des Wirkstoffs und inerten Formkörpern in einer Mischung aus Lösungsmittel und Nicht-Lösungsmittel für den Wirkstoff (a) eine Lösung des Wirkstoffs in dem Lösungsmittel und (b) Nicht-Lösungsmittel zugegeben werden, die Suspension vermischt, bevorzugt gerührt wird, wobei der Wirkstoff aus der Suspension auskristallisiert, der Wirkstoff in Form von Produktpartikeln abgetrennt wird und die Produktpartikel anschließend getrocknet werden, wobei die Produktpartikel kristallin sind, bevorzugt mit einer Kristallintät von ≥ 98 Gew.-%, insbesondere ≥ 99 Gew.-%, in der stabilsten Kristallmodifikation des Wirkstoffs vorliegen, eine Kristalloberfläche von 3 - 10 m²/g aufweisen und die Größenverteilung der Produktpartikel d₅₀ = 1 - 2 µm, d₉₉ < 6 µm und d₁₀₀ < 12 µm beträgt.

Durch das Durchmischen der Suspension werden die in der Suspension enthaltenen Festkörper, d. h. die Wirkstoffpartikel (Primär- und Produktpartikel) und die inerten Formkörper, bewegt. Dies führt zu einer besonders intensiven Durchmischung der Suspension, wodurch eine Zonenbildung mit unterschiedlichen Übersättigungen des Wirkstoffs vermieden wird. Die Konzentration des Wirkstoffs ist in der Flüssigkeit der Suspension daher räumlich im wesentlichen konstant. Eine Ausnahme hiervon stellt die Zugabestelle der Wirkstofflösung dar. Zur Vermeidung hoher Übersättigungen ist es weiter bevorzugt, daß die Wirkstofflösung und das Nicht-Lösungsmittel an unterschiedlichen Stellen zu der Suspension zugegeben werden. Die Zugabe kann auch über Verteilerdüsen, Hohlrührer oder andere, eine schnelle und gleichmäßige Verteilung über das gesamte Suspensionsvolumen unterstützende Vorrichtungen erfolgen. Durch die Vermeidung lokaler Übersättigungsunterschiede wird eine besonders einheitliche Kristallisation und eine enge Partikelgrößenverteilung erreicht.

Das Durchmischen der Suspension kann durch Rühren, Schütteln, Rotieren, Ultraschallbehandlung, Durchleiten von Gasen, Umpumpen der Suspension und andere dem Fachmann bekannte Mischverfahren erfolgen. Bevorzugt ist es, die Suspension zu rühren. Die Durchmischung erfolgt vorzugsweise dergestalt, daß die festen Teilchen (Partikel und Formkörper) in Bewegung gehalten werden. Vorzugsweise bildet sich in der Suspension bei der Durchmischung keine Pfropfenströmung, bei der in axialer und radialer Richtung unterschiedlich gute Durchmischungen stattfinden, sondern es wird die Rührvorrichtung so gewählt, daß eine möglichst intensive und homogene Durchmischung erfolgt, um unterschiedliche Übersättigungen in der Suspension zu vermeiden. Für die sehr enge Partikelgrößenverteilung reicht bei der Verwendung einer Rührvorrichtung der Energieeintrag bei üblichen Rührgeschwindigkeiten in einem Rührreaktor aus. Bevorzugt sind abhängig von der Art und Größe des verwendeten Rührers Rührgeschwindigkeiten von 200-2500 U/min, insbesondere 500-2000 U/min (Umdrehungen pro Minute). Der zugehörige Energieeintrag bei üblichen Rührgeschwindigkeiten ist weit von den in Kugelmühlen oder Hochdruckhomogenisatoren üblichen, spezifischen Energieeinträgen entfernt, wodurch praktisch keine Kontaminationsprobleme durch Abrieb entstehen und die oben geschilderten Nachteile von Zerkleinerungsverfahren vermieden werden, insbesondere werden so Nanoanteile und Amorphisierungen minimiert.

Die Kombination aus eingesetzten Primärpartikeln, inerten Formkörpern und Durchmischung der Suspension führt überraschenderweise dazu, daß die Partikelgröße der Produktpartikel trotz der kristallwachstumsorientierten Fahrweise des erfindungsgemäßen Verfahrens über einen längeren Zeitraum nicht zunimmt, da die über die Produktpartikel abgeführte Kristalloberfläche durch sekundäre Keimbildung ausgeglichen wird, vornehmlich durch die inerten Formkörper befördert. Im Unterschied zu einer keimbildungsorientierten Kristallisation mit hohen Übersättigungen und ohne Primärpartikel ist das erfindungsgemäße Verfahren eine wachstumsorientierte Kristallisation, bei der der Wirkstoff vornehmlich auf bereits vorhandene Kristallflächen kristallisiert.

Aufgrund der Wachstumsorientierung des erfindungsgemäßen Verfahrens ist dieses insbesondere für schlecht bzw. träge kristallisierende Substanzen geeignet, insbesondere solche, die dazu neigen Zwischenformen zu durchlaufen, beispielsweise Öle oder thermodynamische weniger stabile Kristallformen. Im Verlauf des Verfahrens, insbesondere wenn Produktpartikel mehrfach chargenweise oder über einen längeren Zeitraum kontinuierlich entnommen wurden, übernehmen die Produktpartikel die Rolle der zu Anfang des Verfahrens vorliegenden Primärpartikel. Ohne Primärpartikel kann jedoch der Verlauf des Kristallisationsverfahrens und damit die Qualität der Produktpartikel häufig nicht in der gewünschten Weise kontrolliert werden, da ohne die Primärpartikel in der Regel breite Partikelgrößenverteilungen, insbesondere mehr Nanoanteile generiert werden, oft nicht die gewünschte, stabilste Kristallmodifikation erhalten wird und/oder ein großer amorpher Anteil anfällt.

Die Verweilzeit in der Suspension in der Kristallisationsvorrichtung ist bevorzugt >12 min, besonders bevorzugt >15 min und am meisten bevorzugt >20 min. Auf diese Weise werden besonders enge Produktpartikelgrößenverteilungen und geringe Amorphisierungen erhalten. Unter der Verweilzeit wird die durchschnittliche Aufenthaltszeit eines Volumenelements in der Suspension verstanden. Die inerten Formkörper verbleiben vorzugsweise in der Suspension und werden so nicht mit den Produktpartikeln abgetrennt.

Die inerten Formkörper übernehmen in dem erfindungsgemäßen Verfahren unter anderem die Rolle der Förderung einer sekundären Keimbildung, wodurch neue Kristallpartikel entstehen und der Anteil größerer Produktpartikel durch Aufkristallisieren auf vorhandene Partikel (Primär- und Produktpartikel) oder durch Agglomeration in der Suspension minimiert wird. Unter inert wird in diesem Zusammenhang verstanden, daß sich die Formkörper weder im Lösungsmittel für den Wirkstoff noch im Nicht-Lösungsmittel lösen und keinen oder einen nur sehr geringen Abrieb bei der Durchmischung, beispielsweise durch Rühren der Kristallisationssuspension, erzeugen. Die Formkörper können unterschiedliche Formen besitzen, beispielsweise kugel-, ei-, zylinder- oder sternförmig, wobei kugelförmige Formkörper bevorzugt sind. Die Formkörper sind vorzugsweise aus abriebfesten, harten Materialien, wie beispielsweise Zirkonoxid oder Siliciumcarbid. Besonders bevorzugt sind Yttrium-stabilisierte Zirkonoxidkugeln. Die inerten Formkörper weisen eine maximale Ausdehnung von 0,1 - 2 mm auf, bevorzugt 0,2 - 1 mm, beispielsweise können inerte Mikrokugeln mit einem Durchmesser von 0,2 - 1 mm eingesetzt werden. Es hat sich ferner als günstig erwiesen, wenn der Anteil der inerten Formkörper, bezogen auf das Gesamtvolumen der Kristallisationssuspension, etwa 20-80 Vol.-%, bevorzugt 30-70 Vol.-% beträgt. Das Gewichtsverhältnis von Wirkstoffpartikeln (Primär- und Produktpartikel) zu Formkörpern in der Suspension beträgt vorzugsweise 1:1000 bis 1:10, insbesondere 1:200 bis 1:20.

Um die Solventanteile gering zu halten und die Feststoffkonzentration und damit die angebotene Kristallisationsfläche auf hohem Niveau zu halten, kann die Kristallisation mit einer parallel ablaufenden Destillation/Rektifikation gekoppelt werden. Dabei werden die Druckverhältnisse entsprechend der für die Kristallisation optimalen Prozeßtemperatur gewählt. Es ist aber auch möglich, die Suspension nach der Kristallisation durch Eindampfung oder Filtration aufzukonzentrieren.

Das erfindungsgemäße Verfahren kann sowohl diskontinuierlich als auch kontinuierlich durchgeführt werden. Bei einer diskontinuierlichen Verfahrensführung werden die Lösung des Wirkstoffs und das Nicht-Lösungsmittel diskontinuierlich, d.h. chargenweise zugegeben und Produktpartikel chargenweise entnommen, insbesondere in Form einer Suspension von Produktpartikeln. Bevorzugt ist die kontinuierliche Verfahrensführung, bei der eine Lösung des Wirkstoffs und Nicht-Lösungsmittel kontinuierlich zugegeben und eine Suspension von Produktpartikeln kontinuierlich entnommen wird. Die inerten Formkörper verbleiben dabei im Kristallisationsgefäß.

Sowohl bei der diskontinuierlichen als auch bei der kontinuierlichen Verfahrensführung des erfindungsgemäßen Kristallisationsverfahrens ist es bevorzugt, daß das Volumen der Kristallisationssuspension im wesentlichen konstant bleibt, da so eine besonders einheitliche Korngrößenverteilung erreicht werden kann. Unter "im wesentlichen konstant" wird in diesem Zusammenhang verstanden, daß das Volumen der Suspension um nicht mehr als 20 Vol.-% schwankt. Bevorzugt beträgt das Volumen der Suspension, bezogen auf den Mittelwert des Volumens (100 %-Wert), 90-110 Vol.-% insbesondere 95-105 Vol.-%.

Die Lösung des Wirkstoffs kann ein oder mehrere Lösungsmittel für den Wirkstoff umfassen. Geeignete Lösungsmittel sind insbesondere Alkohole, Ketone und Ether, beispielsweise Methanol, Ethanol, Isopropanol, Aceton und Diethylether. Als Nicht-Lösungsmittel kommt bevorzugt Wasser zum Einsatz. Unter einem Nicht-Lösungsmittel wird im Sinne der Erfindung eine Flüssigkeit verstanden, in der sich der Wirkstoff schlecht löst. Die Löslichkeit sollte unter 0,5 g Wirkstoff pro Liter Nicht-Lösungsmittel liegen.

Die Löslichkeit des Wirkstoffs in dem Lösungsmittel beträgt bevorzugt mehr als 10 g/L, die Löslichkeit im Nicht-Lösungsmittel bevorzugt weniger als 0,1 g/L. Besonders bevorzugt beträgt die Löslichkeit des Wirkstoffs in dem Lösungsmittel mehr als 100 g/L, die Löslichkeit im Nicht-Lösungsmittel weniger als 0,1 g/L, weiter bevorzugt weniger als 0,05 g/L. Das Mengenverhältnis von Nicht-Lösungsmittel zu Lösungsmittel ist abhängig von der Löslichkeitsfunktion des Wirkstoffs in dem Lösungsmittel/Nicht-Lösungsmittelsystem. Als günstig hat sich ein Volumenverhältnis von Lösungsmittel : Nicht-Lösungsmittel von 1:1 bis 1:10 erwiesen. Lösungsmittel und Nicht-Lösungsmittel sollten in dem eingesetzten Mengenverhältnis mischbar sein, d. h. eine homogene Mischung bilden können.

Das Trocknen der Produktpartikel kann mit den üblichen Methoden erfolgen, beispielsweise durch Sprühtrocknung, Gefriertrocknung oder andere Arten der Lösungsmittelverdampfung, wobei die Sprühtrocknung bevorzugt ist, insbesondere über eine Düsen- oder Scheibenzerstäubung, da sie die Größe der einzelnen Partikel und damit die Größenverteilung nahezu unbeeinflusst lässt. Die oben beschriebenen Größenverteilungen liegen somit bevorzugt auch bei den getrockneten Produktpartikeln vor.

Der Wirkstoff ist bevorzugt ein Steroid, insbesondere ein Steroidhormon, bevorzugt ein Gestagen oder ein Östrogen, oder ein Antiasthmatikum, bevorzugt ein Glucokortikoid. Unter einem Steroid wird eine Verbindung verstanden, die das Kohlenstoffgerüst des perhydrierten Cyclopenta[a]phenanthrens aufweist. Besonders bevorzugt ist der Wirkstoff gewählt aus der Gruppe bestehend aus Drospirenon, Desogestrel, Dienogest, Ethinylestradiol, Fluticason und Budesonid. Am meisten bevorzugt sind Drospirenon und Fluticason.

Den Produktpartikeln kann vor dem Trocknen ein hydrophiler, pharmazeutisch annehmbarer Hilfsstoff zugesetzt werden, vorzugsweise als Lösung, beispielsweise in Wasser, um eine Wirkstoffpartikel-Festkörperform herzustellen, die die Freisetzung der Wirkstoffpartikel aus der Festkörperform und später aus der Arzneiform bei Kontakt mit Wasser unterstützt. Unter hydrophilen Hilfsstoffen werden Hilfsstoffe verstanden, die in Wasser löslich sind.

Als hydrophile, pharmazeutisch annehmbare Hilfsstoffe kommen vor allem wasserlösliche Hydroxyalkylcellulosen, insbesondere Hydroxyethylcellulose, Hydroxypropylcellulose und Hydroxypropylmethylcellulose, sowie Polyvinylpyrrolidon und ähnliche auch bei der Tablettierung übliche Hilfsstoffe in Betracht. Es können auch weitere Hilfsstoffe zugesetzt werden, beispielsweise die Freisetzung fördernde oder den Wirkstoff schützende Hilfsstoffe. Der Anteil des Hilfsstoffs hängt davon ab, inwieweit die oben beschriebene schnelle Freisetzung der Wirkstoffpartikel aus dem Festkörper erreicht wird. Der Anteil des hydrophilen, pharmazeutisch annehmbaren Hilfsstoffs liegt vorzugsweise zwischen 20% und 90 % Gew.-%, insbesondere zwischen 40% und 75% Gew.-%, bezogen auf die Gesamtmasse der Wirkstoffpartikel-Festkörperform.

Sehr kleine Mikropartikel, vor allem die von hydrophoben Wirkstoffen, zeigen bei Wasserkontakt eine schlechte Benetzung und unter dem Einfluß von van der Waals-Kräften neigen sie zur Bildung von lockeren Agglomeraten, die ein schnelles Auflösen verhindern. Diese Agglomerate können sich unter Umständen auch in der Suspension des erfindungsgemäßen Verfahrens nach Entnahme aus dem Kristallisator bilden, abhängig von Zeit, Restübersättigung und physiko-chemischen Eigenschaften des Wirkstoffs. Daher kann ein möglichst vollständiger Abbau der Übersättigung und eine zügige Weiterverarbeitung erwünscht sein. Deshalb ist eine vorteilhafte Ausgestaltung des Verfahrens eine kontinuierliche Prozessführung, in der unmittelbar nach der kontinuierlichen Entnahme der Produktpartikelsuspension ein hydrophiler Hilfsstoff zugesetzt wird, bevorzugt in Form einer Lösung. Dieser kann beispielsweise über eine Mischstrecke als Hilfsstofflösung zugegeben werden, die mit Hilfsstoff homogen vermischte Suspension einem Rührgefäß zur Pufferung zugeführt und anschließend sprühgetrocknet werden. Hierdurch kann eine etwaige, nachträgliche Agglomeration weitestgehend verhindert werden.

Die Herstellung der Wirkstoffpartikel-Festkörperform erfolgt vorzugsweise durch Sprühtrocknung der Partikelsuspension mit anschließender Pulverabscheidung über einen Produktfilter. Beim Sprühtrocknungsprozeß wird ein Festkörper gebildet, der die Wirkstoffpartikel enthält. Bevorzugt umfasst die Festkörperform hohle Mikropartikel des Hilfsstoffes, an deren Oberfläche wiederum die einzelnen Wirkstoffpartikel eingebettet sind. Hierdurch wird bei Kontakt mit Wasser die sehr schnelle und partikuläre Freisetzung des Wirkstoffskristalle unterstützt, häufig innerhalb von 1-3 Minuten (vgl. Beispiel 3). Damit ist die Voraussetzung für ihre sehr schnelle Auflösung gegeben. In Vitro wurde so nach dem Paddle-Test innerhalb von 5 min eine vollständige Lösung in Wasser bei 37°C und 25% Sättigungskonzentration des Wirkstoffes erreicht.

Die Lösegeschwindigkeit ist dabei weitgehend unabhängig von der Korngröße des Festkörpers. Damit ist die Korngröße lediglich durch die zu erfüllende Gehaltsgleichförmigkeit in der Arzneiform nach oben begrenzt. Die Wirkstoffpartikel-Festkörperform weist bevorzugt eine Größenverteilung mit d₅₀ < 20 µm und d₁₀₀ < 200 µm auf. Die Wirkstoff-Festkörperform kann, wenn dies für die pharmazeutische Applikation vorteilhaft ist, bei Beibehaltung der schnell freisetzenden Eigenschaften, auch mit einem reduzierten Nanoanteil hergestellt werden. Über die Sprühtrocknungstechnologie lassen sich Korngrößen, aber auch weitere Eigenschaften an die pharmazeutische Applikation gut anpassen, zum Beispiel durch Zugabe weiterer Additive, Hilfsstoffe oder Füllmittel.

Die Hilfsstoffpartikel der Wirkstoffpartikel-Festkörperform zerfallen in Wasser bevorzugt innerhalb von 3 min, insbesondere innerhalb von einer Minute. Auf diese Weise gibt die Wirkstoffpartikel-Festkörperform die Wirkstoffpartikel innerhalb von 3 min, insbesondere innerhalb von einer Minute, ohne Aggregation frei. Die Wirkstoffpartikel wiederum lösen sich in Wasser nach ihrer Freisetzung aus der Festkörperform oder in reiner Form nach dem USP-Paddle-Test (Paddle-Test nach der USP-Methode) innerhalb von 10 min zu > 95 Gew.-%, vorzugsweise innerhalb von 5 min zu > 95 Gew.-%.

In einer bevorzugten Ausführungsform setzen daher die kristallinen Wirkstoffpartikel im Paddle-Test nach der USP-Methode mindestens 95 Gew.-% des Wirkstoffes in 10 min, bevorzugt in 5 min, frei. Unter der Freisetzung wird in diesem Zusammenhang verstanden, daß sich der Wirkstoff in Wasser löst. In einer weiteren bevorzugten Ausführungsform setzt die Wirkstoffpartikel-Festkörperform im dem Paddle-Test nach der USP-Methode mindestens 95 Gew.-% des Wirkstoffs in 13 min, bevorzugt in 8 min, besonders bevorzugt in 6 min frei.

Nach der WHO-Definition (Bio Waiver) sollen sehr schnell freisetzende Arzneiformen mindestens 85 % des Wirkstoffs in 15 min freisetzen und schnell freisetzende Arzneiformen mindestens 85 % des Wirkstoffs in 30 min freisetzen. Die kristallinen Wirkstoff-Mikropartikel und die Wirkstoffpartikel-Festkörperform der vorliegenden Erfindung sind daher hervorragend für schnell und insbesondere sehr schnell freisetzende Arzneiformen geeignet.

Das erfindungsgemäße Kristallisationsverfahren kann in einer apparativ einfachen und kostengünstigen Vorrichtung durchgeführt werden. Das Verfahren wird bevorzugt in einem heiz- und kühlbaren Rührwerkskristallisator durchgeführt, der teilweise mit den Formkörpern befüllt ist, die bezüglich der eingesetzten Lösungsmittel, Nicht-Lösungsmittel und Wirkstoffe inert sind. Der Kristallisator kann mit einem Destillations- oder Rektifikationsaufsatz sowie einer Destillatvorlage versehen werden. Weiter umfaßt die Vorrichtung zur Kristallisation Zugabeleitungen für die Wirkstofflösung und das Nicht-Lösungsmittel, sowie eine Entnahmeleitung zur Produktpartikelentnahme. Die Zugabeleitungen können derart ausgeführt sein, daß eine möglichst rasche Verteilung von Wirkstofflösung und Nichtlösungsmittel im Suspensionsvolumen befördert werden, z.B. können Verteilerdüsen vorliegen. Auch der Rührer selbst kann als Zugabeleitung für eine Komponente, z.B. für die Wirkstofflösung, verwendet werden, wenn dieser als Hohlrührer ausgeführt ist und die Flüssigkeit über die Hohlwelle und Rührerblätter direkt in das turbulente Scherfeld des Rührers geleitet wird. Somit erfolgt eine sehr schnelle und homogene Verteilung des gelösten Wirkstoffes über das gesamte Reaktorvolumen und es werden hohe lokale Übersättigungen, die zu Keimbildungsschauern führen können, rasch abgebaut. Die Entnahmeleitung kann mit Filtrationselementen oder Sedimentationszonen für die Rückführung inerter Formkörper in den Rührwerkskristallisator versehen werden. Weiter kann die Entnahmeleitung eine Mischvorrichtung enthalten, beispielsweise einen stationären Mischer, wobei an den Mischer eine weitere Zuleitung angeschlossen werden kann, sofern in dem weiteren Schritt eine Lösung des Hilfsstoffs zugeführt werden soll. Optional kann in der Entnahmeleitung eine Vorrichtung zum Aufkonzentrieren der Suspension enthalten sein, beispielsweise eine Verdampfungs- oder Filtrationseinrichtung. Die Entnahmeleitung führt anschließend zur Trocknungsvorrichtung, die insbesondere eine Sprühtrocknungsanlage ist. Sofern Wirkstoffpartikel-Festkörperformen hergestellt werden sollen, mündet die Entnahmeleitung zunächst in ein Rührgefäß, welches auch als Mischvorrichtung, in welcher der pharmazeutische Hilfsstoff und die Produktpartikel vermischt werden, fungieren kann. Von dort aus führt eine Feedleitung zur Trocknungsvorrichtung, beispielsweise einer Zweistoffdüse oder einer Zerstäubungsscheibe einer Sprühtrockungsanlage.

Beschrieben werden auch kristalline Wirkstoff-Mikropartikel, erhältlich nach dem oben beschriebenen Verfahren. Ferner werden kristalline Wirkstoff-Mikropartikel beschrieben, wobei die Mikropartikel kristallin sind, in der stabilsten Kristallmodifikation vorliegen, eine Kristalloberfläche von 3 - 10 m²/g aufweisen und die Größenverteilung der Mikropartikel d₅₀ = 1 - 2 µm, d₉₉ < 6 µm und d₁₀₀ < 12 µm beträgt. Ferner wird eine Wirkstoff-Festkörperform beschrieben, umfassend einen hydrophilen, pharmazeutisch annehmbaren Hilfsstoff und die oben beschriebenen, kristallinen Wirkstoff-Mikropartikel.

Die oben im Zusammenhang mit dem erfindungsgemäßen Verfahren beschriebenen Ausgestaltungen der Produktpartikel und der Wirkstoffpartikel-Festkörperform gelten ebenso für die kristallinen Wirkstoff-Mikropartikel und die Wirkstoff-Festkörperform. Bevorzugt ist die Kristallinität der Wirkstoff-Mikropartikel größer als die von Mikronisaten. Insbesondere ist die Kristallinität, d.h. der kristalline Anteil ≥ 98 Gew.-%. Bevorzugt ist die Kristallinität der Wirkstoff-Mikropartikel ≥ 99 Gew.-% und besonders bevorzugt ist es, daß die Kristallinität ≥ 99,9 Gew.-% ist.

Ferner wird die Verwendung der beschriebenen kristallinen Wirkstoff-Mikropartikel als Arzneimittel und zur Herstellung von Arzneimitteln beschrieben, bevorzugt für pulmonale Applikationen, sowie die Verwendung der Wirkstoffpartikel-Festkörperform als Arzneimittel und zur Herstellung von Arzneimitteln. Dazu werden die kristallinen Wirkstoff-Mikropartikel oder die Wirkstoffpartikel-Festkörperform gegebenenfalls mit dem Fachmann bekannten Additiven, Hilfsstoffen, Füllstoffen, Schmiermitteln etc. zu einem Arzneimittel verarbeitet.

Für pulmonale Applikationen hängt die Bioverfügbarkeit nicht nur von einer schnellen Auflösung, sondern auch davon ab, daß die Partikelgröße möglichst innerhalb eines bestimmten Bereiches liegt, vorzugsweise zwischen 1 und 5 µm, da ansonsten die Wirkstoffpartikel nicht in Lunge gelangen oder aber wieder ausgeatmet werden. Hier bietet das erfindungsgemäße Verfahren den besonderen Vorteil, daß nicht nur die maximale Korngröße begrenzt, sondern auch der Feinkornanteil im Nanobereich reduziert werden kann.

Bei pulmonal applizierten Wirkstoffen, z.B. Antiasthmatika für Dry Powder Inhaler (DPI) wird der reine Wirkstoff ohne Hilfsstoffe bevorzugt. In diesen Fällen wird daher auf die Zugabe von Hilfsstoffen verzichtet. Durch die Sprühtrocknung, vorzugsweise über Düsenzerstäubung, werden eventuelle lockere Agglomerate zerstört und die feine Primärkörnung des Wirkstoffes wird ohne Vergröberung von der Suspension in den Pulverzustand transformiert. Durch die Begrenzung des Nanoanteils und die hohe Kristallinität des DPI-Wirkstoffes werden zudem Stabilität und Dispergierfähigkeit verbessert.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die nachfolgenden Beispiele erläutern die Erfindung.

### Beispiel 1

In einem 100 ml Rührgefäß mit Blattrührer werden 160 g Y stabilisierte Zirkonoxidkugeln mit einem Durchmesser von 0,5 mm zusammen mit 30 ml Wasser vorgelegt. Zu diesen wird unter Rühren eine Lösung von 1,5 g Drospirenon in 6,25 ml Ethanol/Wasser 4:1 (v/v) zugegeben. Nach Ausfallen einer vorwiegend amorphen Phase beginnt nach etwa 2 min die Umwandlung in die kristalline Modifikation. Diese ist nach 10 bis 15 min abgeschlossen. Nach Ende der Phasentransformation wird innerhalb von 15 min eine Lösung von 4,5 g Drospirenon in 18,75 ml Ethanol Wasser 4:1 (v/v) und in gleichem Verhältnis 90 ml Wasser unter Rühren bei 1500 U/min zugetropft. Gleichzeitig wird bei näherungsweise konstantem Füllstand Produktsuspension abgezogen. Zugabe und Produktabnahme kann ohne nachteiligen Effekt auf das Ergebnis auch quasikontinuierlich in kleineren Portionen erfolgen, wenn der obige Zeitrahmen eingehalten wird. Nach Zugabeende wird zur Vervollständigung der Ausbeute mit ca. 60 ml Wasser nachgespült. Die Suspension im Kristallisator kann aber auch als Startsuspension für den Folgeansatz stehengelassen werden. Insgesamt werden 200 g Suspension mit einem Feststoffgehalt von 25 mg je ml gewonnen. Etwa 15 ml dieser Suspension werden über eine G4 Fritte filtriert und der Feststoff wird an der Luft getrocknet. Von der getrockneten Substanz wird mittels Laserbeugung die Korngrößenverteilung bestimmt (Sympatec Helos, Dispergiersystem Rodos).

Ergebnis: d₁₆ = 0,52 µm, d₅₀ = 1,48 µm, d₉₉ = 4,63 µm, d₁₀₀= 6,00 µm

### Beispiel 2: Sprühtrocknung + Kornanalyse Festköperform

Zu 200 g der wäßrigen Suspension gemäß Beispiel 1 werden 15 g Hydroxypropylmethylcellulose (HPMC) Methocel E5 gegeben und bis zur vollständigen Lösung der HPMC gerührt. Die Suspension wird in einem Laborsprühtrockner Yamato/QVF GA32 mittels Zweistoffdüse unter folgende Bedingungen sprühgetrocknet:
Feed : 4-5 g/min
Trocknungsluft: 0.5 m³/min
Düsendruck : 2.5 bar (ü)
Eintrittstemperatur: 180°C
Austrittstemperatur: 70-75°C

8 g Produktpulver werden in einem Produktfilter abgeschieden

Korngrößenanalyse erfolgte mittels Laserbeugung (Sympatec Helos, Dispergiersystem Rodos):
d₁₆ = 2,0 µm, d₅₀ = 5,2 µm, d₉₉ = 17,4 µm, d₁₀₀= 20,6 µm

### Beispiel 3: Freisetzung Primärpartikel aus Festkörperform (Mikroskopbilder)

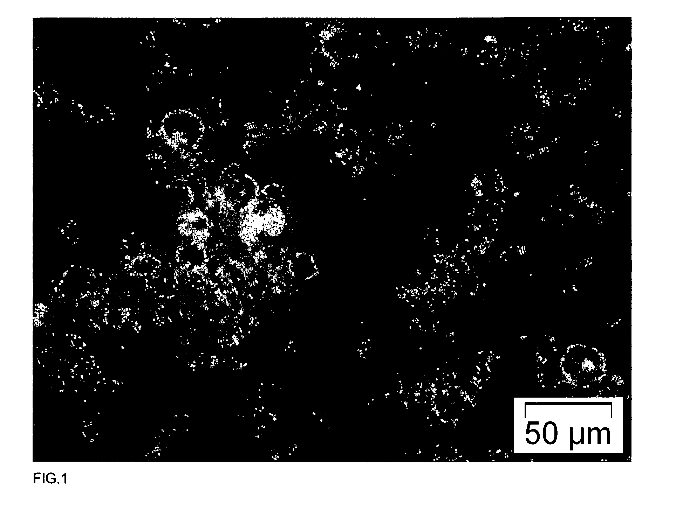

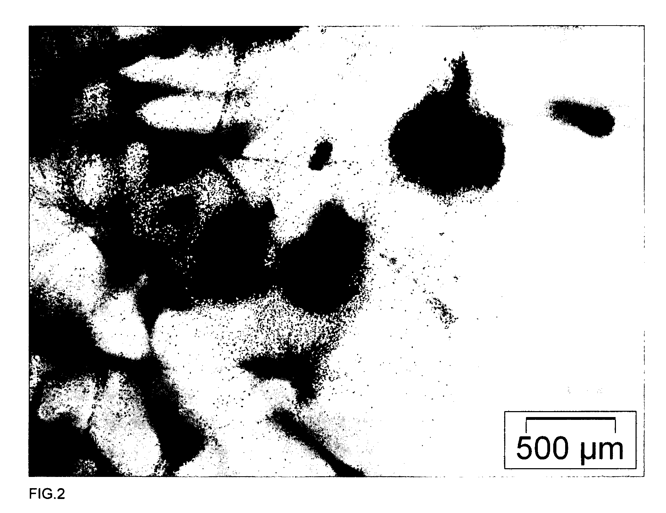

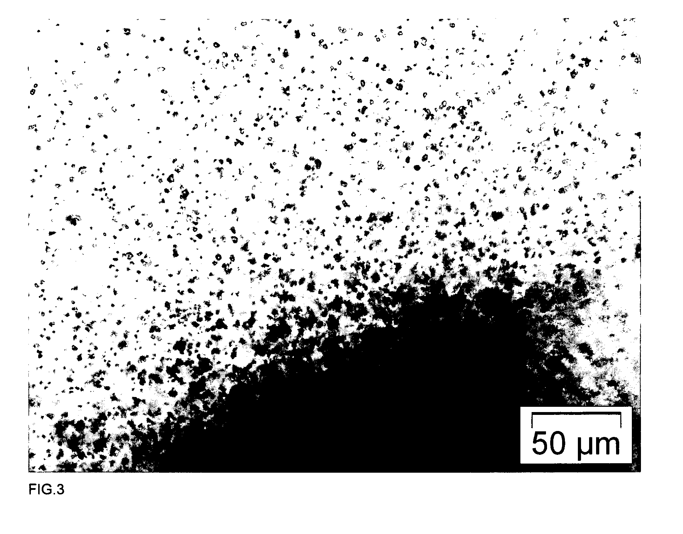

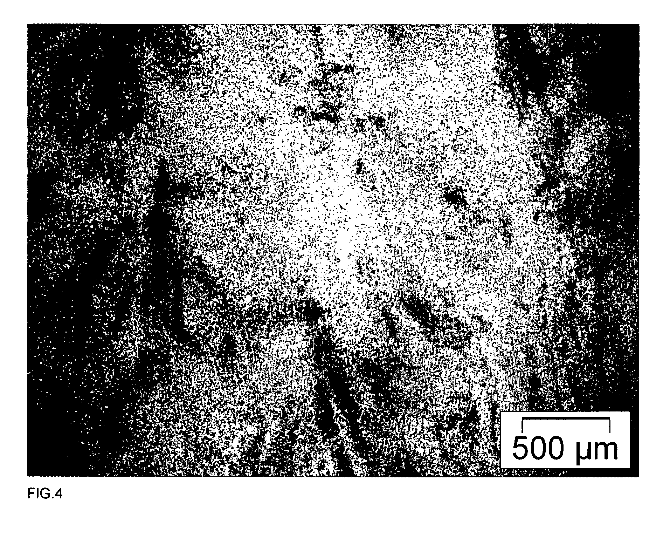

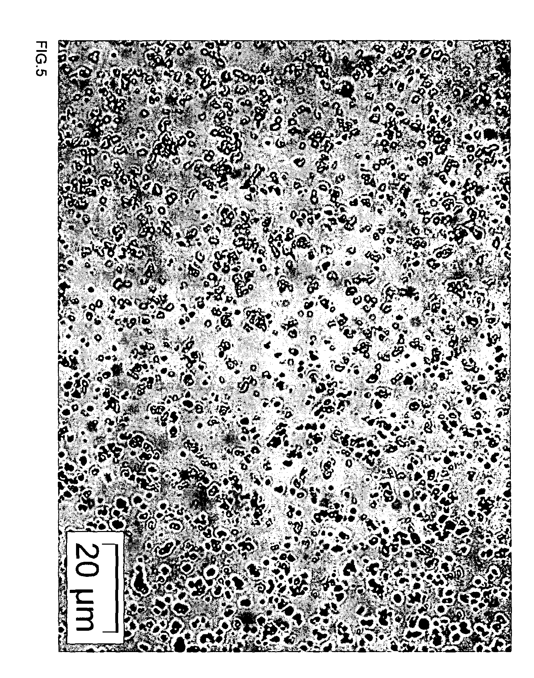

Eine geringe Menge der Festkörperform gemäß Beispiel 2 werden auf eine unbewegte Wasseroberfläche gebracht. Der Zerfallprozeß der Festkörperform wird unter einem Durchlichtmikroskop verfolgt. In FIG.1 ist die sprühgetrocknete Festkörperform abgebildet. An der Oberfläche der kugelförmigen HPMC-Partikell sind die Primärpartikel des Wirkstoffs, unter polarisiertem Licht gut erkennbar, eingebettet. In FIG.2 bis FIG.5 sind die einzelnen Phasen des Freisetzungsprozesses dargestellt. Es wird gezeigt, daß, während der Hilfsstoff in Lösung geht, die Wirkstoffpartikel ohne Aggregation partikulär innerhalb von 1-3 min auf einer unbewegten Wasseroberfläche freigesetzt werden. Dieser Prozeß kann durch Rühren beschleunigt werden.

### Beispiel 4: Löslichkeitskinetik der Festkörperform mit Methocel E5 nach der Paddle-Methode

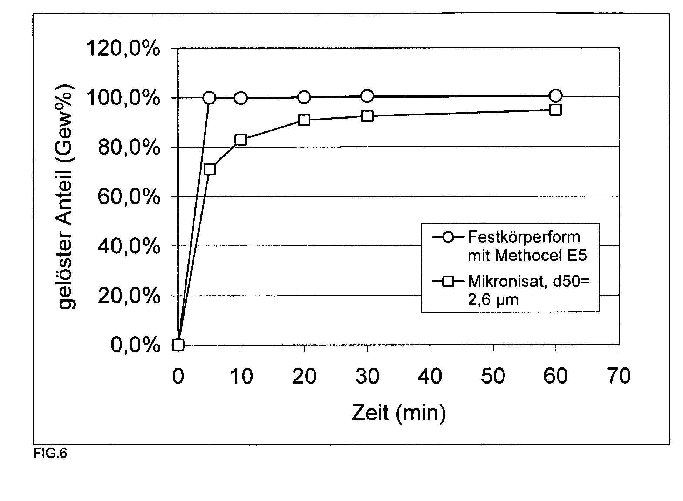

Es werden 14 mg der Festkörperform mit Methocel E5 als Hilfsstoff gemäß Beispiel 2 mit einem Wirkstoffgehalt von 21,5 Gew.-% (entspricht 3 mg Drospirenon) zu 900 ml Wasser, das bei 37°C mit 150 U/min mit einem Blattrührer gerührt wird, gegeben. Es werden eine Blindprobe (Startwert) und Proben bei 5, 10, 20, 30, und 60 min gezogen, über einen 0,2 µm Spritzenvorsatzfilter filtriert und mittels HPLC der gelöste Anteil von Drospirenon ermittelt. Als Vergleich dient ein konventionelles Mikronisat (Strahlmühle). Um ausschließlich das Dissolutionverhalten der Primärkörnung des Mikronisates zu bewerten, müssen Benetzungs- und Agglomerateffekte vermieden werden. Deshalb wurde das Mikronisat vor Zugabe zu den 900 ml Wasser in 2 ml einer PVP-haltigen Lösung dispergiert. Die Ergebnisse der Freisetzung sind in FIG.6 dargestellt. Der Freisetzungsvorgang der Partikel aus der Festkörperform und der anschließende Lösevorgang ist nach 5 min abgeschlossen, während beim konventionellen Mikronisat, selbst bei idealer Benetzung und Dispergierung der Partikel, nach 5 min noch nahezu 30 % des Wirkstoffes ungelöst sind.

### Beispiel 5: Fluticason für pulmonale Anwendung; Kristallisation und Sprühtrocknung ohne Hilfsstoffe

In einem 100 ml Rührgefäß mit Blattrührer werden 160 g Y stabilisierte Zirkonoxidkugeln mit einem Durchmesser von 0,5 mm zusammen mit 30 ml Wasser vorgelegt. Zu diesen wird unter Rühren eine Lösung von 0,6 g Fluticason in 10 ml Aceton zugegeben. Nach 5 min Rühren werden innerhalb von 10 min eine Lösung von 1,8 g Fluticason in 30 ml Aceton und in gleichem Verhältnis 90 ml Wasser unter Rühren bei 1300 U/min zugetropft. Gleichzeitig wird bei näherungsweise konstantem Füllstand Produktsuspension abgezogen. Zugabe und Produktabnahme kann ohne nachteiligen Effekt auf das Ergebnis auch quasikontinuierlich in kleineren Portionen erfolgen, wenn der obige Zeitrahmen eingehalten wird. Nach Zugabeende wird zur Vervollständigung der Ausbeute mit ca. 60 ml Wasser nachgespült. Die Suspension im Kristallisator kann aber auch als Startsuspension für den Folgeansatz stehengelassen werden. Insgesamt werden 180 g Suspension mit einem Feststoffgehalt von 13 mg je ml gewonnen. Die Suspension wird in einem Laborsprühtrockner Yamato/QVF GA32 mittels Zweistoffdüse unter folgenden Bedingungen sprühgetrocknet:
Feed : 6 g/min
Trocknungsluft: 0.3 m³/min
Düsendruck : 3 bar (ü)
Eintrittstemperatur: 170- 180°C
Austrittstemperatur: 65-75°C

1,5 g Produktpulver werden in einem Produktfilter abgeschieden.

Die Korngrößenanalyse erfolgte mittels Laserbeugung (Sympatec Helos, Dispergiersystem Rodos, Dispergierdruck 5 bar).

Ergebnis: d₁₀ = 0,5 µm, d₅₀ = 1,47 µm, d₉₉ = 4,80 µm, d₁₀₀= 6,00 µm

Bestimmung der Kristallinität:
Meßmethode: XRPD (Röntgen Powder Diffraction)
Gerät: Siemens D5000 (feste Probenposition) mit Eulerwiege und Sekundärmonochromator Bragg-Brentano Anordnung
Cu / CuKα-Strahlung
Röhrenspannung: 40 kV
Röhrenstrom 30 mA
28-Bereich: 5-60°C
Schrittweite: 0.03°
Auswertesoftware: EVA Diffrac AT (Bruker AXS)

Kristallinität im Vergeich zum konventionenllen Strahlmühlenmikronisat:
Strahlmühlenmikronisat: 95 Gew.-%
Fluticason: 100 Gew.-%

## Patentansprüche

1. Verfahren zur Herstellung kristalliner Wirkstoff-Mikropartikel, umfassend die Schritte, daß zu einer Suspension von Primärpartikeln eines Wirkstoffs und inerten Formkörpern, mit einer maximalen Ausdehnung von 0,1 - 2 mm, in einer Mischung aus Lösungsmittel und Nicht-Lösungsmittel für den Wirkstoff (a) eine Lösung des Wirkstoffs in dem Lösungsmittel und (b) Nicht-Lösungsmittel gegeben werden, wobei Lösungsmittel und Nicht-Lösungsmittel in dem eingesetzten Mengenverhältnis mischbar sind, die Suspension durchmischt wird, wobei der Wirkstoff aus der Suspension auskristallisiert, der Wirkstoff in Form von Produktpartikeln abgetrennt wird und die Produktpartikel anschließend getrocknet werden, wobei die Primärpartikel und die Produktpartikel kristallin sind, in der stabilsten Kristallmodifikation des Wirkstoffs vorliegen, eine Kristalloberfläche von 3 - 10 m²/g aufweisen und die Größenverteilung der Primär- und Produktpartikel, bestimmt durch Laserbeugung, jeweils d₅₀ = 1 - 2 µm, d₉₉ < 6 µm und d₁₀₀ < 12 µm beträgt, wobei sich diese d-Werte auf Volumenprozente der Partikel beziehen.

2. Verfahren nach Anspruch 1, **dadurch gekennzeichnet, daß** das Abtrennen der Produktpartikel durch Entnehmen einer Suspension von Produktpartikeln erfolgt, vorzugsweise daß das Verfahren kontinuierlich durchgeführt wird.

3. Verfahren nach Anspruch 1 oder 2, **dadurch gekennzeichnet, daß** das Durchmischen durch Rühren der Suspension erfolgt.

4. Verfahren nach einem der Ansprüche 1 bis 3, **dadurch gekennzeichnet, daß** d₁₀ der Produktpartikel < 0,7 µm und d₁₀₀ der Produktpartikel < 8 µm ist.

5. Verfahren nach einem der Ansprüche 1 bis 4, **dadurch gekennzeichnet, daß** die Suspension keine weiteren Hilfsstoffe enthält.

6. Verfahren nach einem der Ansprüche 1 bis 5, **dadurch gekennzeichnet, daß** der Wirkstoff ein Steroidhormon oder ein Glucokortikoid ist, bevorzugt gewählt aus der Gruppe bestehend aus Drospirenon, Desogestrel, Dienogest, Ethinylestradiol, Fluticason und Budesonid.

7. Verfahren zur Herstellung einer Wirkstoffpartikel-Festkörperform, umfassend ein Verfahren gemäß einem der Ansprüche 1 bis 6, bei dem die abgetrennten Produktpartikel vor dem Trocknen zusätzlich mit einem hydrophilen, pharmazeutisch annehmbaren Hilfsstoff versetzt werden.

## Claims

1. Method for producing crystalline active substance microparticles, comprising the steps in which to a suspension of primary particles of active substance and inert formed pieces, with a maximum size of 0.1 - 2 mm, in a mixture of solvent and non-sovent for the active substance (a) a solution of the active substance in the solvent and (b) non-sovent are added, wherein solvent and non-solvent are miscible in the quantitative ratio used, the suspension is mixed, whereupon the active substance crystallizes out of the suspension, the active substance is separated in the form of product particles and the product particles are then dried, wherein the primary particles and the product particles are crystalline, are in the most stable crystal modification of the active substance, have a crystal surface area of 3-10 m²/g and the size distribution of the primary and product particles, determined by laser diffraction, is each d₅₀ = 1-2 µm, d₉₉ < 6 µm and d₁₀₀ < 12 µm, wherein the d values refer to volume percent of the particles.

2. Method according to claim 1, **characterized in that** the separation of the product particles is carried out by removing a suspension of product particles, preferably **in that** the method is carried out continuously.

3. Method according to claim 1 or 2, **characterized in that** mixing is carried out by stirring the suspension.

4. Method according to one of claims 1 to 3, **characterized in that** d₁₀ of the product particles is < 0.7 µm and d₁₀₀ of the product particles is < 8 µm.

5. Method according to one of claims 1 to 4, **characterized in that** the suspension does not contain any further excipients.

6. Method according to one of claims 1 to 5, **characterized in that** the active substance is a steroid hormone or a glucocorticoid, preferably selected from the group comprising drospirenone, desogestrel, dienogest, ethinylestradiol, fluticasone and budesonide.

7. Method for producing a solid form of particles of active substance, comprising a method according to one of claims 1 to 6, in which additionally a hydrophilic, pharmaceutically acceptable excipient is added to the separated product particles prior to drying.

## Revendications

1. Procédé de fabrication de microparticules cristallines d'un principe actif, comprenant les étapes dans lesquelles on ajoute à une suspension de particules primaires d'un principe actif et de corps façonnés inertes, ayant une extension maximale de 0,1-2 mm, dans un mélange constitué d'un solvant et d'un non-solvant pour le principe actif, (a) une solution du principe actif dans le solvant, et (b) le non-solvant, le solvant et le non-solvant étant miscibles selon la proportion pondérale prédéfinie, on mélange intimement la solution, le principe actif se séparant de la suspension par cristallisation, on sépare le principe actif sous forme de particules de produit, puis les particules de produit sont séchées, les particules primaires et les particules de produit étant cristallines, se présentant sous la modification cristalline la plus stable du principe actif, présentant une aire cristalline de 3 à 10 m²/g, et la distribution granulométrique des particules primaires et des particules de produit, déterminée par diffraction laser, étant chacune de d₅₀ = 1-2 µm, d₉₉ < 6 µm et d₁₀₀ < 12 µm, ces valeurs de d étant rapportées au pourcentage en volume des particules.

2. Procédé selon la revendication 1, **caractérisé en ce que** la séparation des particules de produit est réalisée par prélèvement d'une suspension des particules de produit, le procédé étant de préférence mis en oeuvre en continu.

3. Procédé selon la revendication 1 ou 2, **caractérisé en ce que** le mélange intime est réalisé par agitation de la suspension.

4. Procédé selon l'une des revendications 1 à 3, **caractérisé en ce que** le d₁₀ des particules de produit est < 0,7 µm et le d₁₀₀ des particules de produit est < 8 µm.

5. Procédé selon l'une des revendications 1 à 4, **caractérisé en ce que** la suspension ne contient pas d'adjuvants supplémentaires.

6. Procédé selon l'une des revendications 1 à 5, **caractérisé en ce que** le principe actif est une hormone stéroïdienne ou un glucocorticoïde, de préférence choisi dans le groupe consistant en la drospirénone, le désogestrel, le diénogest, l'éthinylestradiol, la fluticasone et le budésonide.

7. Procédé de fabrication d'une forme solide de particules de principe actif, comprenant un procédé selon l'une des revendications 1 à 6, dans lequel les particules de produit séparées sont, avant le séchage, encore additionnées d'un adjuvant hydrophile, pouvant être éliminé par voie pharmaceutique.
